# EUROPEAN PATENT APPLICATION

(11) **EP 1 609 480 A1**
(43) Date of publication of application: **28.12.2005**
(21) Application number: 04725524.5
(22) Date of filing: 02.04.2004
(51) Int. Cl.: A61K 45/00, A61K 31/5575, A61P 25/00

(54) **REMEDY FOR SPINAL CANAL STENOSIS**

(30) Priority: 03.04.2003 JP 2003100388
(71) Applicant: ONO PHARMACEUTICAL CO., LTD., Osaka-shi, Osaka 541-8526 (JP)
(72) Inventor: Takenobu, Yoshifumi, Ono Pharmaceutical Co., Ltd., Mishima-gun, Osaka 618-8585 (JP); Kamanaka, Yoshihisa, Ono Pharmaceutical Co., Ltd., Mishima-gun, Osaka 618-8585 (JP); Obata, Takaaki, Ono Pharmaceutical Co., Ltd., Mishima-gun, Osaka 618-8585 (JP)
(74) Representative: Henkel, Feiler & Hänzel
(86) International application number: PCT/JP2004/004836
(87) International publication number: WO 2004/089411

(57) **Abstract**

A remedy for spinal canal stenosis which comprises a combination of a compound having EP2 agonism with a compound having EP3 agonism. A drug comprising a combination of a compound having EP2 agonism with a compound having EP3 agonism shows an efficacy in a rat gait disorder model induced by compression of cauda equina. Namely, it is efficacious against spinal canal stenosis to use a combination of a compound having EP2 agonism with a compound having EP3 agonism or a compound having both EP2 agonism and EP3 agonism.

## Description

### TECHNICAL FIELD

The present invention relates to an agent for treating spinal canal stenosis. More specifically, the present invention relates to an agent for preventing and/or treating spinal canal stenosis which has EP2 agonist action and EP3 agonist action.

### BACKGROUND ART

The internal space enclosed with each vertebral body and processus spinalis from the cervical vertebra to the sacral vertebra is called spinal canal. The spinal canal stenosis presents various symptoms by narrowing spinal canal due to the hypertrophic degeneration of the spine, one of constituents of spinal canal, and the yellow ligament and due to the projection of the intervertebral disc *etc*., followed by compressing the nerve tissue of the nerve root and the cauda equina *etc*. The spinal canal stenosis is classified into the wide spinal canal stenosis, the thoracic spinal canal stenosis and the lumbar spinal canal stenosis, by narrow parts of spinal canal. Their symptoms by the nerve compression are lumbar pain, upper or lower limbs pain, and numbness, *etc*. Especially, when the cauda equina nerve is injured, lumbar pain, lower limbs pain, numbness and languidness deteriorate. This symptom is called an intermittent claudication.

PGE₂ has been known as a metabolite in the arachidonate cascade. It has been known that PGE2 possesses cyto-protective activity, uterine contractive activity, a pain-inducing effect, a promoting effect on digestive peristalsis, an awakening effect, a suppressive effect on gastric acid secretion, hypotensive activity and diuretic activity and so on.

A recent study has proved existence of various PGE₂ subtype receptors possessing a different physiological or pharmacological role from each other. At present, four receptor subtypes are known and they are called EP1, EP2, EP3, and EP4 (Negishi M., *et al., J. Lipid Mediators Cell Signaling,* 12, 379-391 (1995)). Discovery of a drug which is useful for various diseases with less side effects made possible by using a compound which mainly binds to these subtype receptors selectively to thereby examine the function of each of these subtype receptors.

It is described that a compound represented by the following formula (I) has EP2 agonist action and is useful for prevention and/or treatment of immune diseases (autoimmune diseases, organ transplantation *etc*.), asthma, abnormal bone formulation, neuron cell death, liver damage, abortion, premature birth or retinal neuropathy such as glaucoma *etc*. (EP860430A1).

It is described that a compound represented by the following formula (II) has EP3 agonist action and is useful for prevention and/or treatment of liver diseases, Kidney disease, pancreatitis or myocardial infarction *etc.* (WO98/34916).

It is described that a compound represented by the following formula (III) has EP2 agonist action and is useful for prevention and/or treatment of immune diseases, allergic diseases, neuronal cell death, dysmenorrhea, premature birth, abortion, baldness, retinal neuropathy, erectile dysfunction, arthritis, pulmonary injury, pulmonary fibrosis, pulmonary emphysema, bronchitis, chronic obstructive pulmonary disease, hepatic injury, acute hepatitis, liver cirrhosis, shock, nephritis, renal failure, circulatory diseases, systemic inflammatory response syndrome, sepsis, hemophagocytosis syndrome, macrophage activation syndrome, still disease, Kawasaki Disease, burn, systemic granuloma, ulcerative colitis, Crohn disease, hypercytokinemia at dialysis, multiple organ failure, or bone diseases *etc.* (WO2003/74483).

### DISCLOSURE OF THE INVENTION

As the therapeutic method for spinal canal stenosis, in the case of critical condition, a surgical treatment by surgical orthopedics is selected, whereas, in the case of slight conditions, the basic therapeutic method is a conservative treatment, including gymnastic therapy for reinforcing muscle such as abdominal muscle or muscle of back; thermotherapy such as hot pack; acupuncture for ease of the pain; brace therapy such as application of corset, *etc*.; and the like. Also, in the case of slight or mild conditions, a drug treatment is used alone or in combination with the above various treatments. Many of spinal canal stenosis are treated by the conservative treatment, and there are many cases in which the symptom is improved by a combination of various conservative treatments. A drug for spinal canal stenosis in the drug treatment which has been recently approved is only an oral preparation of prostaglandin E1 derivative used for the improvement of blood circulation in the nerve tissue. However, a drug for satisfactorily improving various symptoms in motor function, nerves and others in spinal canal stenosis has not been found yet.

The present inventors studied eagerly in order to find a novel therapeutic agent for spinal canal stenosis, and as a result, surprisingly, found that a medicament having EP2 agonist action and EP3 agonist action improves symptoms of spinal canal stenosis, and thus the present invention has been completed. Treatment of spinal canal stenosis by a combination of an EP2 agonist and EP3 agonist has not been known until now. The present inventors demonstrated, for the first time, the therapeutic effect on spinal canal stenosis by a combination of an EP2 agonist and an EP3 agonist using a gait disorder model induced by compression of cauda equina (*J. Neurosci. Methods,* 104(2) 191-198 (2002)), and has completed the present invention.

The present invention relates to
1. an agent for preventing and/or treating spinal canal stenosis which comprises a combination of a compound having EP2 agonist action and a compound having EP3 agonist action,
2. the agent for preventing and/or treating spinal canal stenosis according to claim 1, wherein the compound having EP2 agonist action and the compound having EP3 agonist action are each administrated,
3. the agent for preventing and/or treating spinal canal stenosis according to claim 1, wherein the compound having EP2 agonist action and the compound having EP3 agonist action are comprised in the same preparation,
4. the agent for preventing and/or treating spinal canal stenosis according to claim 1, wherein the compound having EP2 agonist action is a compound represented by formula (I) 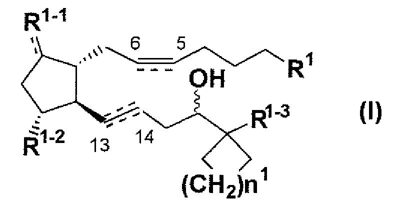 wherein R¹ is carboxy or hydroxymethyl; R¹⁻¹ is oxo, methylene or a halogen atom; R¹⁻² is a hydrogen atom, hydroxy or C1-4 alkoxy; R¹⁻³ is a hydrogen atom, C1-8 alkyl, C2-8 alkenyl, C2-8 alkynyl, or C1-8 alkyl, C2-8 alkenyl or C2-8 alkynyl substituted by 1-3 of substituents selected from the following (1) to (5): (1) a halogen atom, (2) C1-4 alkoxy, (3) C3-7 cycloalkyl, (4) phenyl or (5) phenyl substituted by 1-3 of substituents selected from a halogen atom, C1-4 alkyl, C1-4 alkoxy, nitro or trifluoromethyl; n¹ is 0 or 1-4; 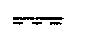 is a single bond or a double bond; 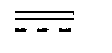 is a double bond or a triple bond; 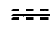 is a single bond, a double bond or a triple bond; 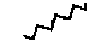 is α-configuration, β-configuration or a mixture of them,
   a salt thereof, a solvate thereof or a prodrug thereof, or a cyclodextrin clathrate thereof,
5. the agent for preventing and/or treating spinal canal stenosis according to claim 1, wherein the compound having EP3 agonist action is a compound represented by formula (II) 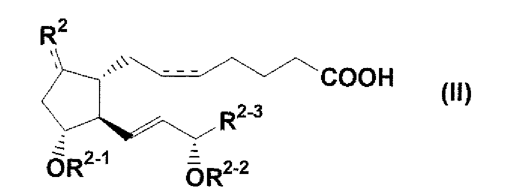 wherein R² is oxo or a halogen atom; R²⁻¹ and R²⁻² are each independently C1-4 alkyl; R²⁻³ is C1-10 alkyl, C2-10 alkenylene, C2-10 alkynylene, or C1-10 alkyl, C2-10 alkenylene, C2-10 alkynylene substituted by phenyl, phenoxy, C3-7 cycloalkyl or C3-7 cycloalkyloxy, in which the phenyl and the cycloalkyl may be substituted by 1-3 of C1-4 alkyl, C1-4 alkoxy, a halogen atom, trihalomethyl or nitro; 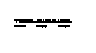 is a single bond or a double bond,
   a salt thereof, a solvate thereof or a prodrug thereof, or cyclodextrin clathrate thereof,
6. the agent for preventing and/or treating spinal canal stenosis according to claim 1, wherein the compound having EP2 agonist action is a compound represented by formula (III) 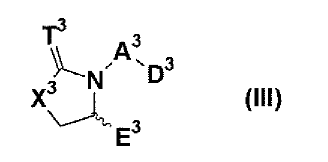 wherein T³ is (1) an oxygen atom or (2) a sulfur atom;
   X³ is (1) -CH₂-, (2) -O- or (3) -S-;
   A³ is A³⁻¹ or A³⁻²; A³⁻¹ is (1) C2-8 straight-chain alkylene optionally substituted by 1 to 2 C1-4 alkyl, (2) C2-8 straight-chain alkenylene optionally substituted by 1 to 2 C1-4 alkyl or (3) C2-8 straight-chain alkynylene optionally substituted by 1 to 2 C1-4 alkyl; A³⁻² is -G³⁻¹-G³⁻²-G³⁻³-; G³⁻¹ is (1) C1-4 straight-chain alkylene optionally substituted by 1 to 2 C1-4 alkyl, (2) C2-4 straight-chain alkenylene optionally substituted by 1 to 2 C1-4 alkyl or (3) C2-4 straight-chain alkynylene optionally substituted by 1 to 2 C1-4 alkyl; G³⁻² is (1) -Y³-, (2) -ring 1³-, (3) -Y³-ring 1³-, (4) -ring 1³-Y³- or (5) -Y³-C1-4 alkylene-ring 1³-; Y³ is (1) -S-, (2) -SO-, (3) -SO₂-, (4) - O- or (5) -NR³⁻¹-; R³⁻¹ is (1) a hydrogen atom, (2) C1-10 alkyl or (3) C2-10 acyl; G³⁻³ is (1) a bond, (2) C1-4 straight-chain alkylene optionally substituted by 1 to 2 C1-4 alkyl, (3) C2-4 straight-chain alkenylene optionally substituted by 1 to 2 C1-4 alkyl or (4) C2-4 straight-chain alkynylene optionally substituted by 1 to 2 C1-4 alkyl;
   D³ is D³⁻¹ or D³⁻²; D³⁻¹ is (1) -COOH, (2) -COOR³⁻², (3) tetrazol-5-yl or (4) - CONR³⁻³SO₂R³⁻⁴; R³⁻² is (1) C1-10 alkyl, (2) phenyl, (3) C1-10 alkyl substituted by phenyl or (4) biphenyl; R³⁻³ is (1) a hydrogen atom or (2) C1-10 alkyl; R³⁻⁴ is (1) C1-10 alkyl or (2) phenyl; D³⁻² is (1) -CH₂OH, (2) -CH₂OR³⁻⁵, (3) hydroxy, (4) -OR³⁻⁵, (5) formyl, (6) -CONR³⁻⁶R³⁻⁷, (7) -CONR³⁻⁶SO₂R³⁻⁸, (8) -CO-(NH-amino acid residue-CO)ₘ₃-OH, (9)-O-(CO-amino acid residue-NH)ₘ₃-H, (10) -COOR³⁻⁹, (11) -OCO-R³⁻¹⁰, (12) -COO-Z³⁻¹-Z³⁻²-Z³⁻³ or 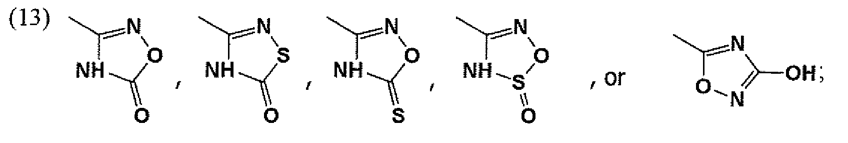 R³⁻⁵ is C1-10 alkyl; R³⁻⁶ and R³⁻⁷ are each independently (1) a hydrogen atom or (2) C1-10 alkyl; R³⁻⁸ is C1-10 alkyl substituted by phenyl; R³⁻⁹ is (1) C1-10 alkyl substituted by biphenyl optionally substituted by 1 to 3 substituents selected from C1-10 alkyl, C1-10 alkoxy and a halogen atom or (2) biphenyl substituted by 1 to 3 substituents selected from C1-10 alkyl, C1-10 alkoxy and a halogen atom; R³⁻¹⁰ is (1) phenyl or (2) C1-10 alkyl; m³ is 1 or 2; Z³⁻¹ is (1) C1-15 alkylene, (2) C2-15 alkenylene or (3) C2-15 alkynylene; Z³⁻² is (1) -CO-, (2) -OCO-, (3) -COO-, (4) -CONR^{Z3-1}-, (5) -NR^{Z3-2}CO-, (6) -O-, (7) -S-, (8) -SO₂-, (9) -SO₂-NR^{Z3-2}-, (10) -NR^{Z3-2}SO₂-, (11) -NR^{Z3-3}-, (12) -NR^{Z3-4}CONR^{Z3-5}-, (13) -NR^{Z3-6}COO-, (14) -OCONR^{Z3-7}- or (15) -OCOO-; Z³⁻³ is (1) a hydrogen atom, (2) C1-15 alkyl, (3) C2-15 alkenyl, (4) C2-15 alkynyl, (5) ring Z³ or (6) C1-10 alkyl substituted by C1-10 alkoxy, C1-10 alkylthio, C1-10 alkyl-NR^{Z3-8}- or ring Z³; ring Z³ is (1) C3-15 mono-, bi- or tri-carbocyclic aryl which may be partially or fully saturated or (2) 3 to 15 membered mono-, bi- or tri-heterocyclic aryl containing 1 to 4 hetero atoms selected from oxygen, nitrogen and sulfur atom which may be partially or fully saturated; R^{Z3-1}, R^{Z3-2}, R^{Z3-3}, R^{Z3-4}, R^{Z3-5}, R^{Z3-6}, R^{Z3-7} and R^{Z3-8} are each independently a hydrogen atom or C1-15 alkyl, R^{Z3-1} and Z³⁻³ may be taken together with the nitrogen atom to which they are attached to form 5 to 7 membered saturated mono-heterocyclic ring, and the heterocyclic ring may contain other one hetero atom selected from oxygen, nitrogen and sulfur atoms, ring Z³ and the saturated mono-heterocyclic ring formed by R^{Z3-1}, Z³⁻³ and the nitrogen atom to which they are attached may be substituted by 1-3 groups selected from following (1) to (4); (1) C1-15 alkyl, (2) C2-15 alkenyl, (3) C2-15 alkynyl, (4) C1-10 alkyl substituted by C1-10 alkoxy, C1-10 alkylthio or C1-10 alkyl-NR^{Z3-9}-; R^{Z3-9} is a hydrogen atom or C1-10 alkyl, 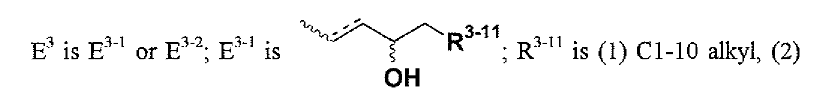 C1-10 alkylthio, (3) C1-10 alkyl substituted by C3-8 cycloalkyl, (4) C1-10 alkyl substituted by ring 2 or (5) C1-10 alkyl substituted by -W³⁻¹-W³⁻²-ring 2; W³⁻¹ is (1) -O-, (2) -S-, (3) -SO-, (4) -SO₂-, (5) -NR³⁻¹¹⁻¹-, (6) carbonyl, (7) -NR³⁻¹¹⁻¹SO₂-, (8) carbonylamino or (9) aminocarbonyl; R³⁻¹¹⁻¹ is (1) a hydrogen atom, (2) C1-10 alkyl or (3) C2-10 acyl; W³⁻² is (1) a bond or (2) C1-8 alkyl optionally substituted by C1-4 alkyl, a halogen atom or hydroxy; E³⁻² is (1) U³⁻¹-U³⁻²-U³⁻³ or (2) ring 4³; U³⁻¹ is (1) C1-4 alkylene, (2) C2-4 alkenylene, (3) C2-4 alkynylene, (4) -ring 3³-, (5) C1-4 alkylene-ring 3³-, (6) C2-4 alkenylene-ring 3³- or (7) C2-4 alkynylene-ring 3³-; U³⁻² is (1) a bond, (2) -CH₂-, (3) -CHOH-, (4) -O-, (5) -S-, (6) -SO-, (7) -SO₂-, (8) -NR³⁻¹²-, (9) carbonyl, (10) -NR³⁻¹²SO₂-, (11) carbonylamino or (12) aminocarbonyl; R³⁻¹² is (1) a hydrogen atom, (2) C1-10 alkyl or (3) C2-10 acyl; U³⁻³ is (1) C1-8 alkyl optionally substituted by 1 to 3 substituents selected from C1-10 alkyl, a halogen atom, hydroxy, alkoxy, alkylthio and NR³⁻¹³R³⁻¹⁴, (2) C2-8 alkenyl optionally substituted by 1 to 3 substituents selected from C1-10 alkyl, a halogen atom, hydroxy, alkoxy, alkylthio and NR³⁻¹³R³⁻¹⁴, (3) C2-8 alkynyl optionally substituted by 1 to 3 substituents selected from C1-10 alkyl, a halogen atom, hydroxy, alkoxy, alkylthio and NR³⁻¹³R³⁻¹⁴, (4) C1-8 alkyl substituted by ring 4³ or (5) ring 4³; R³⁻¹³ and R³⁻¹⁴ are each independently (1) a hydrogen atom or (2) C1-10 alkyl; ring 1³, ring 2³, ring 3³ or ring 4³ may be substituted by 1 to 5 of R³; R³ is (1) C1-10 alkyl, (2) C2-10 alkenyl, (3) C2-10 alkynyl, (4) C1-10 alkoxy, (5) C1-10 alkylthio, (6) a halogen atom, (7) hydroxy, (8) nitro, (9) -NR³⁻¹⁵R³⁻¹⁶, (10) C1-10 alkyl substituted by C1-10 alkoxy, (11) C1-10 alkyl substituted by 1 to 3 halogen atoms, (12) C1-10 alkyl substituted by C1-10 alkoxy substituted by 1 to 3 halogen atoms, (13) C1-10 alkyl substituted by -NR³⁻¹⁵R³⁻¹⁶, (14) ring 5³, (15) -O-ring 5³, (16) C1-10 alkyl substituted by ring 5³, (17) C2-10 alkenyl substituted by ring 5³, (18) C2-10 alkynyl substituted by ring 5³, (19) C1-10 alkoxy substituted by ring 5³, (20) C1-10 alkyl substituted by -O-ring 5³, (21) COOR³⁻¹⁷, (22) C1-10 alkoxy substituted by 1 to 4 halogen atom, (23) formyl, (24) C1-10 alkyl substituted by hydroxy or (25) C2-10 acyl, R³⁻¹⁵; R³⁻¹⁶ and R³⁻¹⁷ are each independently (1) a hydrogen atom or (2) C1-10 alkyl; ring 5³ may be substituted by 1 to 3 substituents selected from following (1)-(9); (1) C1-10 alkyl, (2) C2-10 alkenyl, (3) C2-10 alkynyl, (4) C1-10 alkoxy, (5) C1-10 alkyl substituted by C1-10 alkoxy, (6) a halogen atom, (7) hydroxy, (8) C1-10 alkyl substituted by 1 to 3 halogen atoms, (9) C1-10 alkyl substituted by C1-10 alkoxy substituted by 1 to 3 halogen atoms; ring 1³, ring 2³, ring 3³, ring 4³ and ring 5³ are each independently (1) C3-15 mono-, bi- or tri-carbocyclic aryl which may be partially or fully saturated or (2) 3 to 15 membered mono-, bi- or tri-heterocyclic aryl containing hetero atoms selected from 1 to 4 nitrogen, 1 to 2 oxygen and/or 1 to 2 sulfur atom which may be partially or fully saturated; 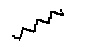 is α-conliguration, β-configuration or mixture of them,
   a salt thereof, a solvate thereof or a prodrug thereof, or cyclodextrin clathrate thereof,
7. an agent for preventing and/or treating spinal canal stenosis which comprises a compound having EP2 agonist action and EP3 agonist action,
8. the agent for preventing and/or treating spinal canal stenosis according to claim 1 or 7, wherein the spinal canal stenosis is cervical spinal canal stenosis, thoracic spinal canal stenosis, lumbar spinal canal stenosis or wide spinal canal stenosis,
9. the agent for preventing and/or treating spinal canal stenosis according to claim 1 or 7, which is an agent for improving paralysis, hypoesthesia, pain or numbness,
10. the agent for preventing and/or treating spinal canal stenosis according to claim 1 or 7, which is an agent for improving physical ability,
11. the agent for preventing and/or treating spinal canal stenosis according to claim 10, wherein the improving physical ability is improving muscle weakness, intermittent claudication or ambulatory ability,
12. the agent for preventing and/or treating spinal canal stenosis according to claim 1 or 7, which is an agent for treating bladder trouble or rectum trouble,
13. a medicament which comprises a combination of the agent for preventing and/or treating spinal canal stenosis according to claim 1 or 7 and one or more medicaments selected from prostaglandins, prostaglandin derivatives formulations, nonsteroidal anti-inflammatory drugs, vitamins, muscle relaxants, antidepressants, poly ADP-ribose polymerase inhibitors, excitatory amino acid receptor antagonists, radical scavengers, astrocyte modulators, IL-8 receptor antagonists, immunosuppressive drugs, nitric oxide synthase inhibitor and aldose reductase inhibitors,
14. a method for preventing and/or treating spinal canal stenosis in a mammal, which comprises administering to a mammal an effective amount of a compound having EP2 agonist action and a compound having EP3 agonist action, or a compound having EP2 agonist action and EP3 agonist action, and
15. use of a compound having EP2 agonist action and a compound having EP3 agonist action, or a compound having EP2 agonist action and EP3 agonist action for preparation of an agent for preventing and/or treating spinal canal stenosis.

The agent for preventing and/or treating spinal canal stenosis of the present invention includes any compound which has EP2 agonist action and/or EP3 agonist action.

The compound which has EP2 agonist action and/or EP3 agonist action include a compound having EP2 agonist action, a compound having EP3 agonist action, or a compound having EP2 agonist action and EP3 agonist action. In addition, not only the compound having EP2 agonist action and/or EP3 agonist action that has known but also the one that will be newly found in the future are included. The compounds having EP2 agonist action include, for example, compounds described in EP860430A1, compounds described in WO99/33794, compounds described in EP974580A1, compounds described in WO95/19964, compounds described in US5698598, compounds described in US6376533, compounds described in WO98/28264, compounds described in WO99/19300, compounds described in EP0911321A1, compounds described in WO98/58911, compounds described in WO2003/74483, AH-13205, CP-533536, butaprost, lioprost, misoprostol or AY23626 etc.

Preferred as the compound having EP2 agonist action is, for example, the above compound represented by formula (I).

In formula (I), C1-4 alkyl means, for example, methyl, ethyl, propyl, butyl and the branched isomers thereof.

C1-8 alkyl means, for example, methyl, ethyl, propyl, butyl, pentyl, hexyl, heptyl, octyl and the branched isomers thereof.

C2-8 alkenyl means, for example, vinyl, propenyl, butenyl, pentenyl, hexenyl, heptenyl, octenyl and the branched isomers thereof.

C2-8 alkynyl means, for example, ethynyl, propynyl, butynyl, pentynyl, hexynyl, heptynyl, octynyl and the branched isomers thereof.

C1-4 alkoxy means, for example, methoxy, ethoxy, propoxy, butoxy and the branched isomers thereof.

C3-7 cycloalkyl means, for example, cyclopropyl, cyclobutyl, cyclopentyl, cyclohexyl and cycloheptyl *etc*.

A halogen atom means, for example, fluoride, chloride, bromide and iodide atom.

In the compound represented by formula (I), more preferred is, for example, (5Z,9β,11α,13E)-17,17-propano-11,16-dihydroxy-9-chloro-20-norprosta-5,13-dienoic acid or lysine salt thereof *etc*.

Moreover, preferred as the compound having EP2 agonist action is, for example, the above compound represented by formula (III).

In formula (III), C1-4 alkyl means, for example, methyl, ethyl, propyl, butyl and the isomers thereof.

In formula (III), C1-8 alkyl means, for example, methyl, ethyl, propyl, butyl, pentyl, hexyl, heptyl, octyl and the isomers thereof.

C1-10 alkyl means, for example, methyl, ethyl, propyl, butyl, pentyl, hexyl, heptyl, octyl, nonyl, decyl and the isomers thereof.

C1-15 alkyl means, for example, methyl, ethyl, propyl, butyl, pentyl, hexyl, heptyl, octyl, nonyl, decyl, undecyl, dodecyl, tridecyl, tetradecyl, pentadecyl and the isomers thereof.

C2-8 alkenyl means, for example, ethenyl, propenyl, butenyl, pentenyl, hexenyl, heptenyl, octenyl and the isomers thereof.

C2-10 alkenyl means, for example, ethenyl, propenyl, butenyl, pentenyl, hexenyl, heptenyl, octenyl, nonenyl, decenyl and the isomers thereof.

C2-15 alkenyl means, for example, ethenyl, propenyl, butenyl, pentenyl, hexenyl, heptenyl, octenyl, nonenyl, decenyl, undecenyl, dodecenyl, tridecenyl, tetradecenyl, pentadecenyl and the isomers thereof.

C2-8 alkynyl means ethynyl, propynyl, butynyl, pentynyl, hexynyl, heptynyl, octynyl and the isomers thereof.

C2-10 alkynyl means, for example, ethynyl, propynyl, butynyl, pentynyl, hexynyl, heptynyl, octynyl, nonynyl, decynyl and the isomers thereof.

C2-15 alkynyl means, for example, ethynyl, propynyl, butynyl, pentynyl, hexynyl, heptynyl, octynyl, nonynyl, decynyl, undecynyl, dodecynyl, tridecynyl, tetradecynyl, pentadecynyl and the isomers thereof.

C1-4 straight-chain alkylene means, for example, methylene, ethylene, trimethylene and tetramethylene.

C2-8 straight-chain alkylene means, for example, ethylene, trimethylene, tetramethylene, pentamethylene, hexamethylene, heptamethylene and octamethylene.

C1-4 alkylene means, for example, methylene, ethylene, trimethylene, tetramethylene and the isomers thereof.

C1-15 alkylene means, for example, methylene, ethylene, trimethylene, tetramethylene, pentamethylene, hexamethylene, heptamethylene, octamethylene, nonamethylene, decamethylene, undecamethylene, dodecamethylene, tridecamethylene, tetradecamethylene, pentadecamethylene and the isomers thereof.

C2-4 straight-chain alkenylene means, for example, ethenylene, propenylene, butenylene and the isomers thereof.

C2-8 straight-chain alkenylene means C2-8 alkenylene which has 1 to 2 double bond(s). It means, for example, ethenylene, propenylene, butenylene, butadienylene, pentenylene, pentadienylene, hexenylene, hexadienylene, heptenylene, heptadienylene, octenylene and octadienylene.

C2-4 alkenylene means, for example, ethenylene, propenylene, butenylene and the isomers thereof.

C2-15 alkenylene means, for example, ethenylene, propenylene, butenylene, pentenylene, hexenylene, heptenylene, octenylene, nonenylene, decenylene, undecenylene, dodecenylene, tridecenylene, tetradecenylene, pentadecenylene and the isomers thereof.

C2-4 straight-chain alkynylene means, for example, ethynylene, propynylene and butynylene.

C2-8 straight-chain alkynylene means C2-8 alkynylene which has 1 to 2 triple bond(s). It means, for example, ethynylene, propynylene, butynylene, butadiynylene, pentynylene, pentadiynylene, hexynylene, hexadiynylene, heptynylene, heptadiynylene, octynylene and octadiynylene.

C2-4 alkynylene means, for example, ethynylene, propynylene, butynylene and the isomers thereof.

C2-15 alkynylene means, for example, ethynylene, propynylene, butynylene, pentynylene, hexynylene, heptynylene, octynylene, nonynylene, decynylene, undecynylene, dodecynylene, tridecynylene, tetradecynylene, pentadecynylene and the isomers thereof.

C1-10 alkoxy means, for example, methoxy, ethoxy, propoxy, butoxy, pentyloxy, hexyloxy, heptyloxy, octyloxy, nonyloxy, decyloxy and the isomers thereof.

C1-10 alkylthio means, for example, methylthio, ethylthio, propylthio, butylthio, pentylthio, hexylthio, heptylthio, octylthio, nonylthio, decylthio and the isomers thereof.

C3-8 cycloalkyl means, for example, cyclopropyl, cyclobutyl, cyclopentyl, cyclohexyl, cycloheptyl and cyclooctyl.

C2-10 acyl means, for example, ethanoyl, propanoyl, butanoyl, pentanoyl, hexanoyl, heptanoyl, octanoyl, nonanoyl, decanoyl and the isomers thereof

Biphenyl means, for example, 2-phenylphenyl, 3-phenylphenyl or 4-phenylphenyl.

Halogen atom means, for example, fluoride, chloride, bromide and iodide atom.

Amino acid residue in -CO-(NH- amino acid residue-CO)ₘ₃-OH and -O-(CO-amino acid residue-NH)ₘ₃-H means the amino acid residue of natural amino acid or abnormal amino acid. Natural amino acids or abnormal amino acid include, for example, glycine, alanine, valine, leucine, isoleucine, serine, threonine, cystein, methionine, proline, asparagine, glutamine, phenylalanine, tyrosine, tryptophan, aspartic acid, glutamic acid, lysine, arginine, histidine, β-alanine, cystathionine, cystine, homoserine, isoleucine, lanthionine, norleucine, norvaline, ornithine, sarcosine, thyronine *etc*.

In amino acid residue in -CO-(NH- amino acid residue-CO)ₘ₃-OH and -O-(CO-amino acid residue-NH)ₘ₃-H, an amino acid with protecting group is included.

In formula (III), C3-15 mono-, bi- or tri-carbocyclic aryl which may be partially or fully saturated represented by ring1³, ring2³, ring3³, ring4³, ring5³ or ringZ³ includes, for example, cyclopropane, cyclobutane, cyclopentane, cyclohexane, cycloheptane, cyclooctane, cyclononane, cyclodecane, cycloundecane, cyclododecane, cyclotridecane, cyclotetradecane, cyclopentadecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indane, naphthalene, dihydronaphthalene, tetrahydronaphthalene, perhydronaphthalene, heptalene, perhydroheptalene, biphenylene, as-indacene, s-indacene, acenaphthylene, acenaphthene, fluorene, phenalene, phenanthrene, anthracene, spiro[4.4]nonane, spiro[4.5]decane, spiro[5.5]undecane, bicyclo[2.2.1]heptane, bicyclo[2.2.1]hept-2-ene, bicyclo[3.1.1]heptane, bicyclo[3.1.1]hept-2-ene, bicyclo[2.2.2]octane, bicyclo[2.2.2]oct-2-ene, adamantane, noradamantane *etc*.

Among the 3 to 15 membered mono-, bi- or tri-heterocyclic aryl containing hetero atoms selected from 1 to 4 nitrogen, 1 to 2 oxygen, and/or 1 to 2 sulfur atoms which may be partially or fully saturated represented by ring1³, ring2³, ring3³, ring4³, ring5³ or ringZ³, 3 to 15 membered mono-, bi- or tri-heterocyclic aryl containing hetero atoms selected from 1 to 4 nitrogen, 1 to 2 oxygen, and/or 1 to 2 sulfur atoms includes, for example, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzoxepine, benzoxazepine, benzoxadiazepine, benzothiepine, benzothiazepine, benzothiadiazepine, benzazepine, benzodiazepine, benzofurazan, benzothiadiazole, benzotriazole, carbazole, beta-carboline, acridine, phenazine, dibenzofuran, xanthene, dibenzothiophene, phenothiazine, phenoxazine, phenoxathiin, thianthrene, phenanthridine, phenanthroline, perimidine ring *etc.*

The 3 to 15 membered mono-, bi- or tri-heterocyclic aryl containing hetero atoms selected from 1 to 4 nitrogen, 1 to 2 oxygen and/or 1 to 2 sulfur atoms which is partially or fully saturated includes aziridine, azetidine, azocane, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, dioxaindan, benzodioxane, chroman, benzodithiolane, benzodithiane ring *etc*.

C3-10 mono- or bi-carbocyclic aryl which may be partially or fully saturated includes, for example, cyclopropane, cyclobutane, cyclopentane, cyclohexane, cycloheptane, cyclooctane, cyclononane, cyclodecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indane, naphthalene, dihydronaphthalene, tetrahydronaphthalene, perhydronaphthalene, spiro[4.4]nonane, spiro[4.5]decane, bicyclo[2.2.1]heptane, bicyclo[2.2.1]hept-2-ene, bicyclo[3.1.1]heptane, bicyclo[3.1.1]hept-2-ene, bicyclo[2.2.2]octane, bicyclo[2.2.2]oct-2-ene, adamantane, noradamantane *etc*.

Among the 3 to 10 membered mono- or bi-heterocyclic aryl containing hetero atoms selected from 1 to 4 nitrogen, 1 to 2 oxygen, and/or 1 to 2 sulfur atoms which may be partially or fully saturated, 3 to 10 membered mono- or bi-heterocyclic aryl containing hetero atoms selected from 1 to 4 nitrogen, 1 to 2 oxygen, and/or 1 to 2 sulfur atoms includes, for example, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, rurazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzofurazan, benzothiadiazole, benzotriazole ring *etc*.

The 3 to 10 membered mono- or bi-heterocyclic aryl containing hetero atoms selected from 1 to 4 nitrogen, 1 to 2 oxygen and/or 1 to 2 sulfur atoms which is partially or fully saturated includes aziridine, azetidine, azocane, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroi sob enzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dioxolane, dioxane, dithiolane, dithiane, dioxaindan, benzodioxane, chroman, benzodithiolane, benzodithiane ring *etc*.

C3-7 mono-carbocyclic aryl which may be partially or fully saturated includes, for example, cyclopropane, cyclobutane, cyclopentane, cyclohexane, cycloheptane, cyclopentene, cyclohexene, cycloheptene, cyclopentadiene, cyclohexadiene, cycloheptadiene, benzene *etc*.

Among the 3 to 7 membered mono-heterocyclic aryl containing hetero atoms selected from to 4 nitrogen, 1 to 2 oxygen, and/or 1 to 2 sulfur atoms which may be partially or fully saturated, 3 to 7 membered mono-heterocyclic aryl containing hetero atoms selected from 1 to 4 nitrogen, 1 to 2 oxygen, and/or 1 to 2 sulfur atoms includes, for example, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine ring *etc.*

The 3 to 7 membered mono-heterocyclic aryl containing hetero atoms selected from 1 to 4 nitrogen, 1 to 2 oxygen and/or 1 to 2 sulfur atoms which is partially or fully saturated includes aziridine, azetidine, azocane, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, dioxolane, dioxane, dithiolane, dithiane ring *etc.*

C5 or 6 mono-carbocyclic aryl which may be partially or fully saturated includes, for example, cyclopentane, cyclohexane, cyclopentene, cyclohexene, benzene *etc.*

Among the 5 or 6 membered mono-heteracyclic aryl containing hetero atoms selected from 1 to 4 nitrogen, 1 to 2 oxygen, and/or 1 to 2 sulfur atoms which may be partially or fully saturated, 5 or 6 membered mono-heterocyclic aryl containing hetero atoms selected from 1 to 4 nitrogen, 1 to 2 oxygen, and/or 1 to 2 sulfur atoms includes, for example, pyrrole, imidazole, triazole, tetrazole, pyrazole, pyridine, pyrazine, pyrimidine, pyridazine, furan, pyran, thiophene, oxazole, isoxazole, thiazole, isothiazole, furazan, oxadiazole, oxazine, oxadiazine, thiadiazole, thiazine, thiadiazine ring *etc*.

The 5 or 6 membered mono-heterocyclic aryl containing hetero atoms selected from 1 to 4 nitrogen, 1 to 2 oxygen and/or 1 to 2 sulfur atoms which is partially or fully saturated includes pyrroline, pyrrolidine, imidazoline, imidazolidine, triazoline, triazolidine, tetrazoline, tetrazolidine, pyrazoline, pyrazolidine, piperidine, piperazine, morpholine, thiomorpholine, oxathiane, dioxolane, dioxane, dithiolane, dithiane ring *etc.*

Among the 3 to 15 membered mono-, bi- or tri-heterocyclic aryl containing at least one nitrogen atom and opptionally 1 to 2 oxygen, and/or 1 to 2 sulfur atoms which may be partially or fully saturated, 3 to 15 membered mono-, bi- or tri-heterocyclic aryl containing at least one nitrogen atom and opptionally 1 to 2 oxygen, and/or 1 to 2 sulfur atoms includes, for example, pyrrole, imidazole, triazole, tetrazole, pyrazole, indazole, purine, benzimidazole, benzazepine, benzotriazole, carbazole, β-carboline, phenothiazine, phenoxazine, perimidine *etc*.

The 3 to 15 membered mono-, bi- or tri-heterocyclic aryl containing at least one nitrogen atom and optionally 1 to 2 oxygen, and/or 1 to 2 sulfur atoms which is partially or fully saturated includes aziridine, azetidine, azocane, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, indoline, isoindoline, dihydroindazole, perhydroindazole, dihydroquinoline, tetrahydroquinoline, perhydroquinoline, dihydroisoquinoline, tetrahydroisoquinoline, perhydroisoquinaline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, dihydrobenzoxazepine, tetrahydrobenzoxazepine, dihydrocarbazole, tetrahydrocarbazole, perhydrocarbazole, dihydroacridine, tetrahydroacridine, perhydroacridine *etc.*

Among the 5 to 7 membered mono-heterocyclic aryl containing at least one nitrogen atom and opptionally 1 to 2 oxygen, and/or 1 to 2 sulfur atoms which may be partially or fully saturated, 5 to 7 membered mono-heterocyclic aryl containing at least one nitrogen atom and opptionally 1 to 2 oxygen, and/or 1 to 2 sulfur atoms includes, for example, pyrrole, imidazole, triazole, tetrazole, pyrazole *etc.*

The 5 to 7 membered mono-heterocyclic aryl containing at least one nitrogen atom and opptionally 1 to 2 oxygen, and/or 1 to 2 sulfur atoms which is partially or fully saturated includes pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine *etc*.

In the compound represented by formula (III), more preferred is, for example, 2-[(2-{(2R)-2-[(1E,4S)-4-(1-ethylcyclobutyl)-4-hydroxy-1-butenyl]-5-oxo-1-pyrrolidinyl}ethyl)sulfanyl]-1,3-thiazole-4-carboxylic acid or 2-[(2-{(2R)-2-[(3,5-dichlorophenoxy)meyhyl]-5-oxo-1-pyrrolidinyl}ethyl)sulfanyl]-1,3-thiazol-4-carboxylic acid *etc.*

The compounds having EP3 agonist action include, for example, compounds described in WO98/34916, compounds described in JP 07-233145, compounds described in JP 10-168056, compounds described in JP 11-012249, compounds described in WO99/25358, compounds described in JP 7-215929, compounds described in JP 8-239356, compounds described in WO97/05091, TEI-3356, M&B-28767, GR63799X, SC-46275, enprostil, sulprostone *etc*.

Preferred as the compound having EP3 agonist action is the above compound represented by formula (II).

In formula (II), C1-4 alkyl means, for example, methyl, ethyl, propyl, butyl and the branched isomers thereof

C1-4 alkoxy means, for example, methoxy, ethoxy, propoxy, butoxy and the branched isomers thereof

C1-10 alkyl means, for example, methyl, ethyl, propyl, butyl, pentyl, hexyl, heptyl, octyl, nonyl, decyl and the branched isomers thereof.

C2-10 alkenyl means, for example, vinyl, propenyl, butenyl, pentenyl, hexenyl, heptenyl, octenyl, nonenyl, decenyl and the branched isomers thereof.

C2-10 alkynyl means, for example, ethynyl, propynyl, butynyl, pentynyl, hexynyl, heptynyl, octynyl, nonynyl, decynyl and the branched isomers thereof.

C3-7 cycloalkyl means, for example, cyclopropyl, cyclobutyl, cyclopentyl, cyclohexyl or cycloheptyl.

A halogen atom means, for example, fluoride, chloride, bromide and iodide atom.

Among the compounds represented by formula (II), more preferred is 11α,15α-dimethoxy-9-oxoprosta-5Z,13E-dienoic acid *etc.*

Moreover, the compound having EP2 agonist action and EP3 agonist action is included in the present invention.

The compound used in the present invention includes salts prepared by known methods. Preferred salt is a pharmacologically acceptable salt.

The pharmacologically acceptable salt is for example, alkaline metal, alkaline earth metal, ammonium salt or amine salt in the case where a parent compound is an acidic compound, and for example organic or inorganic acid addition salt in the case where a parent compound is a basic compound.

The pharmacologically acceptable salt is preferably water-soluble. The suitable salt means, for example, salt of alkaline metal (potassium, sodium, *etc.),* salt of alkaline earth metal (calcium, magnesium, *etc*.), ammonium salt, pharmaceutically acceptable salt of organic amine or amino acid (tetramethylammonium, triethylamine, methylamine, dimethylamine, cyclopentylamine, benzylamine, phenethylamine, piperidine, monoethanolamine, diethanolamine, tris(hydroxymethyl)aminomethane, lysine, arginine, N-methyl-D-glucamine, *etc*.).

The acid addition salt is preferably water-soluble. The suitable acid addition salt means, for example, inorganic acid salt (hydrochloride, hydrobromate, hydroiodate, sulfate, phosphate, nitrate, *etc.),* or organic acid salt (acetate, lactate, tartrate, benzoate, citrate, methane sulfonate, ethane sulfonate, benzene sulfonate, toluene sulfonate, isethionate, glucuronate, gluconate, *etc.), etc.*

The compound used in the present invention and the salt thereof may be converted solvate.

The solvate is preferably non toxic and water-soluble. The suitable solvate is, for example, solvate of water or alcohol (e.g. ethanol).

Unless otherwise specified, plane structural and stereochemical isomers are included in the present invention. In the former, for example, alkyl, alkoxy, alkylthio group includes straight or branched ones. In the latter case addition, isomers on double bond, ring, fused ring (E-, Z-, cis-, trans-isomer), isomers generated from asymmetric carbon atom(s) (R-, S-, α-, β-isomer, enantiomer, diastereomer), optically active isomers (D-, L-, d-, 1-isomer) are included. In addition, polar compounds generated by chromatographic separation (more polar compound, less polar compound), equilibrium compounds, mixtures thereof at voluntary ratios and racemic mixtures are also included in the present invention.

Moreover, the compounds used in the present invention may be prodrugs prepared by known methods.

According to the present invention, unless otherwise indicated and as is apparent for those skilled in the art, symbol 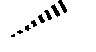 indicates that it is bound to the opposite side of the sheet (namely α-configuration), symbol 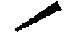 indicates that it is bound to the front side of the sheet (namely β-configuration), symbol 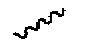 indicates that it is α-configuration, β-configuration or a mixture thereof, and symbol 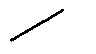 indicates that it is a mixture of α-configuration and β-configuration.

A prodrug of the compound used in the present invention means a compound which is converted to the compound used in the present invention by reaction with an enzyme, gastric acid or the like in the living body. For example, with regard to a prodrug of the compound used in the present invention, when the compound used in the present invention has a hydroxyl group, compounds where the hydroxyl group is, for example, acylated, alkylated, phosphorylated or borated (e.g., compounds in which the hydroxyl group of the compound used in the present invention is acetylated, palmitoylated, propanoylated, pivaloylated, succinylated, fumarylated, alanylated or dimethylaminomethylcarbonylated); and that the carboxyl group of the compound used in the present invention is, for example, esterified or amidated (e.g., compounds in which the carboxyl group of the compound used in the present invention is made into ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester or methylamide). Those compounds may be produced by a known method *per se*. The prodrug of the compound used in the present invention may be either a hydrate or a non-hydrate.

The compounds used in the present invention or the esters thereof may be converted into the corresponding cyclodextrin clathrates by the method described in the specification of GB1,351,238 or GB1,419,221 using α-, β- or γ-cyclodextrin or a mixture thereof. Converting into the corresponding cyclodextrin clathrates serves to increase the stability and solubility in water of the compounds, and therefore it is useful in the use for pharmaceuticals.

### [Processes for the preparation of the compound used in the present invention]

The compound used in the present invention, the prodrug thereof or the salt thereof can be prepared by known methods or methods described in the specification of EP860430A1, WO99/33794, EP974580A1, WO95/19964, US5698598, US6376533, WO98/28264, WO99/19300, EP0911321A1, WO98/58911, WO2003/74483, WO98/34916, JP-A-7-233145, JP-A-10-168056, JP-A-11-012249, WO99/25358, JP-A-7-215929, JP-A-8-239356 or WO97/05091.

### Toxicity:

It has been confirmed that the compounds used in the present invention have low toxicity and are sufficiently safe for use as pharmaceutical preparations. For example, the maximal tolerated dose of the compound A (lysine salt of (5Z,9β,11α,13E)-17,17-propano-11,16-dihydroxy-9-chloro-20-norprosta-5,13-dienoic acid) in the compound represented by formula (I) used in the present invention was 30 mg/kg weight or more for rat intravenous administration.

### INDUSTRIAL APPLICABILITY

### Application to pharmaceuticals

As described later, and administering the combination of the compound having EP2 agonist action and the compound having EP3 agonist action are effective in a gait disorder model induced by compression of cauda equina known as a spinal canal stenosis model. Therefore, a medicament having EP2 agonist action and EP3 agonist action is useful for spinal canal stenosis, and can improve physical ability, especially muscle weakness, intermittent claudication or ambulatory ability. Furthermore, the compound is useful for paralysis, hypoesthesia, pain, or numbness of patients, especially lower limb paralysis, hypoesthesia, pain or numbness. In addition, it is effective in the therapy for bladder or rectum disorder due to spinal canal stenosis. Bladder disorder due to spinal canal stenosis means dysuria due to it. It includes frequent urination, delayed urination, forceless urinary stream, ischuria and urinary incontinence. Furthermore, rectal disorder due to spinal canal stenosis means defecation disorder due to it.

The effect of treatment for spiral canal stenosis by the compound used in the present invention is thought to be based on the improvement of hypofunction of the surrounding tissue of spinal canal, for example intervertebral disk; the improvement of hyperplasia of yellow ligament, posterior ligament or the like; the improvement of inflammation or reduction of blood flow due to nerve compression; or the nerve protection.

In the present invention, at least one compound having EP2 agonist action and at least one compound having EP3 agonist action may be administered as separate preparations, that is, in the form of combined administration. The combined administration includes simultaneous administration and administration with time difference. In the administration with time difference, the compound having EP2 agonist action may be administered in advance, and then the compound having EP3 agonist action may be administered. Alternatively, the compound having EP3 agonist action may be administered in advance, and then the compound having EP2 agonist action may be administered. The administration methods of the respective compounds are the same or different. Also, at least one compound having EP2 agonist action and at least one compound having EP3 agonist action may be included in the same preparation. Furthermore, a compound having both EP2 agonist action and EP3 agonist action may be singly administered. Moreover, at least one compound having EP2 agonist action and at least one compound having both EP2 agonist action and EP3 agonist action, or at least one compound having EP3 agonist action and at least one compound having both EP2 agonist action and EP3 agonist action may be administered in combination. Additionally, at least one compound having EP2 agonist action, at least one compound having EP3 agonist action and at least one compound having both EP2 agonist action and EP3 agonist action may be administered in combination.

The medicaments of the present invention may be administered as a combined preparation by combining with other medicaments for the purpose of
1) supplementing and/or enhancing of prevention and/or treatment effect of the compound,
2) improvement in pharmacokinetics and absorption and reduction of dose of the compound, and/or
3) reduction of side effect of the compound.

The combined preparation of the medicaments of the present invention with other medicaments may be administered in a form of a compounded agent in which both components are compounded in a preparation or may be in a form in which they are administered by means of separate preparations. The case of administration by means of separate preparations includes a simultaneous administration and administrations with time difference. In the case of administrations with time difference, the medicament of the present invention may be firstly administered followed by administering the other medicament or the other medicament may be administered firstly followed by administering the medicament of the present invention.

### Methods for each of the administration are the same or different.

If necessary, other medicaments *etc*. may be combined for supplementing and/or enhancing the treatment effect for spiral canal stenosis of the medicament of the present invention includes. The other medicaments include, for example, prostaglandins, prostaglandin derivatives formulations, nonsteroidal anti-inflammatory drugs (NSAID), vitamins, muscle relaxants, antidepressants, poly ADP-ribose polymerase (PARP) inhibitors, excitatory amino acid receptor antagonists (such as NMDA receptor antagonists and AMPA receptor antagonists), radical scavengers, astrocyte modulators, IL-8 receptor antagonists, immunosuppressive drugs (such as cyclosporine and FK506), nitric oxide synthase inhibitor and aldose reductase inhibitors.

Examples of prostaglandins (hereinafter, abbreviated as PG) include PG receptor agonists, PG receptor antagonists, and the like. Examples of PG receptors include PGE receptors (EP1, EP2, EP3 and EP4), PGD receptors (DP, CRTH2), PGF receptors (FP), PGI receptors (IP), TX receptors (TP), and the like. In addition, examples of prostaglandin derivative formulations include limaprost, beraprost and the like.

Examples of NSAID include sasapyrine, sodium salicylate, aspirin, aspirin dialuminate, diflunisal, indomethacin, suprofen, ufenamate, dimethylisopropylazulene, bufexamac, felbinac, diclofenac, tolmetin sodium, clinoril, fenbufen, nabumetone, proglumetacin, indomethacin farnesyl, acemetacin, proglumetacin maleate, amfenac sodium, mofezolac, etodolac, ibuprofen, ibuprofen piconol, naproxen, flurbiprofen, flurbiprofen axetil, ketoprofen, Fenoprofen calcium salt, tiaprofenic acid, oxaprozin, pranoprofen, loxoprofen sodium, alminoprofen, zaltoprofen, mefenamic acid, mefenamic acid aluminium, tolfenamic acid, floctafenine, ketophenylbutazone, oxiphenbutazone, piroxicam, tenoxicam, ampiroxicam, napageln ointment, epirizole, tiaramide hydrochloride, tinoridine hydrochloride, emorfazone, sulpvrine, migrenin, Saridon, Sedes G, amipylo-N, solvon, pyrine compounding cold medicine, acetaminophen, phenacetin, dimetotiazine mesilate, cimetoride-combined drug, non-pyrine-combined cold medicine and the like.

Examples of muscle relaxants include tolperisone hydrochloride, chlorzoxazone, chlormezanone, methocarbamol, phenprobamate, pridinol mesilate, chlorphenesin carbamate, baclofen, eperisone hydrochloride, afloqualone, tizaindine hydrochloride, alcuronium chloride, suxamethonium chloride, tubocurarine chloride, dantrolene sodium, pancuronium bromide, vecuronium bromide and the like.

Examples of tricyclic antidepressants include imipramine hydrochloride, desipramine hydrochloride, clomipramine hydrochloride, trimipramine maleate, amitriptyline hydrochloride, nortriptyline hydrochloride, lofepramine hydrochloride, amoxapine, dosulepin hydrochloride and the like.

Examples of tetracyclic antidepressants include maprotiline, mianserin and the like.

Examples of aldose reductase inhibitors include epalrestat, fidarestat, AS-3201, zenarestat, imirestat, AL-4114, ICI-10552, ICI-215918, ZD-5522, BAL-ARI8, methosorbinil, FR-62765, WF-2421, GP-1447, IDD-598, JTT-811, ADN-138, ADN-311, lindolrestat, SG-210, M-16049, M-16209, NZ-314, sorbinil, zopolrestat, CP-AR-3192, ascorbyl gamolenate, risarestat, salfredins, AD-5467, TJN-732, TAT, tolrestat, thiazocin-A, axillarin, ICI-215918, ponalrestat, minalrestat, DN-108, SPR-210, A-74863a and the like.

The amount used of the medicament of the present invention and the other medicament is not especially limited. If it is an amount safely used, any amount is acceptable.

Arbitrary two or more of the other medicaments may be administered in combination.

Moreover, examples of the other medicaments for supplementing and/or enhancing the treatment effect of the medicaments of the present invention include not only known compounds but also new compound.

The other medicament may be any preparation generally used. For example, solid compositions (tablets, pills, capsules, dispersible powders and granules *etc.)* and liquid compositions (solutions, suspensions, emulsions, syrups and elixirs *etc*.) *etc*. are included.

In order to use the medicaments of the present invention or the medicaments of the present invention in combination with the other medicaments, these are normally administered to the entire or local part of human body orally or parenterally.

The doses to be administered are determined depending upon, for example, age, body weight, symptom, the desired therapeutic effect, the route of administration, and the duration of the treatment as well as the medicament used in the invention. In the human adult, the doses per person are generally from 1 µg to 100 mg, by oral administration, up to several times per day, and from 0.1 ng to 10 mg, by parenteral administration, up to several times per day, or continuous administration from 1 to 24 hours per day from vein.

As mentioned above, the doses depend upon various conditions. Therefore, there are cases in which doses lower than or greater than the ranges specified above may be used.

The medicaments of the present invention, or concomitant medication combined the medicaments with other medicaments may be administered in the composition of, for example, solid compositions or liquid compositions, each for oral administration, or injections, external use, suppositories, inhalant or nasal spray each for parenteral administration.

Examples of the solid preparations for internal use for oral administration include tablets, pills, capsules, powders, granules and the like. The capsules include hard capsules and soft capsules. The tablets include sublingual tablets, intraoral patches, orally fast disintegrating tablets and the like.

Such a solid preparation for internal use is prepared by a formulation method commonly employed by using one or two or more active substances either as it is or as a mixture with an excipient (lactose, mannitol, glucose, microcrystalline cellulose, starch, *etc*.), a binder (hydroxypropylcellulose, polyvinylpyrrolidone, magnesium metasilicate aluminate, *etc.),* a disintegrating agent (calcium cellulose glycolate, *etc*.), a lubricant (magnesium stearate, etc.), a stabilizer and a dissolution aid (glutamic acid, aspartic acid, *etc.).* If necessary, it may be coated with a coating agent (sucrose, gelatin, hydroxypropylcellulose, hydroxypropylmethylcellulose phthalate, *etc.).* It may be coated with two or more layers. Moreover, capsules made of an absorbable material such as gelatin are involved in the scope thereof:

The sublingual tablets may be prepared in accordance with a well known method. For example, a sublingual tablet is prepared by a formulation method commonly employed by using one or more active substances are used mixed with an excipient (lactose, mannitol, glucose, microcrystalline cellulose, starch, *etc.),* a binder (hydroxypropylcellulose, polyvinylpyrrolidone, magnesium metasilicate aluminate, *etc*.), a disintegrator (starch, L-hydroxypropyl cellulose, carboxymethyl cellulose, croscarmellose sodium, calcium cellulose glycolate, *etc*.), a lubricant (magnesium stearate, *etc.),* a swelling agent (hydroxypropyl cellulose, hydroxylpropylmethy cellulose, carbopol, carboxymethyl cellulose, polyvinyl alcohol, xanthan gum, guar gum, *etc*.), a swelling aid agent (glucose, fructose, mannitol, xylitol, erythritol, maltose, trehalose, phosphate, citrate, silicate, glycine, glutamic acid, arginine, *etc*.), a stabilizer and a dissolution aid (polyethylene glycol, propylene glycol, glutamic acid, aspartic acid, *etc*.), a flavoring agent (orange, strawberry, mint, lemon, vanilla, *etc*.). If necessary, it may be coated with a coating agent (sucrose, gelatin, hydroxypropylcellulose, hydroxypropylmethylcellulose phthalate, *etc*.). If necessary, it may be coated with two or more layers. Moreover, it may also further comprise some additives such as sweetening agents, antioxidants, coloring agents, preservatives and the like.

The intraoral patch may be prepared in accordance with a well known method. For example, a intraoral patch is prepared by a formulation method commonly employed by using one or more active substances are used mixed with an excipient (lactose, mannitol, glucose, microcrystalline cellulose, starch, *etc*.), a binder (hydroxypropylcellulose, polyvinylpyrrolidone, magnesium metasilicate aluminate, *etc*.), a disintegrator (starch, L-hydroxypropyl cellulose, carboxymethyl cellulose, croscarmellose sodium, calcium cellulose glycolate, *etc*.), a lubricant (magnesium stearate, *etc.*), a attach agent (hydroxypropyl cellulose, hydroxylpropylmethy cellulose, carbopol, carboxymethyl cellulose, polyvinyl alcohol, xanthan gum, guar gum, *etc.),* a attach aid agent (glucose, fructose, mannitol, xylitol, erythritol, maltose, trehalose, phosphate, citrate, silicate, glycine, glutamic acid, arginine, *etc*.), a stabilizer and a dissolution aid (polyethylene glycol, propylene glycol, glutamic acid, aspartic acid, *etc.),* a flavoring agent (orange, strawberry, mint, lemon, vanilla, *etc.)* and the like. If necessary, it may be coated with a coating agent (sucrose, gelatin, hydroxypropylcellulose, hydroxypropylmethylcellulose phthalate, *etc*.) and the like. If necessary, it may be coated with two or more layers. Moreover, it may also further comprise some additives such as sweetening agents, antioxidants, coloring agents, preservatives and the like.

Liquid forms for oral administration include pharmaceutically acceptable solutions, suspensions and emulsions, syrups and elixirs. In such forms, one or more of the active compound(s) may be dissolved, suspended or emulsified into diluent(s) commonly used in the art (such as purified water, ethanol or a mixture thereof). Besides such liquid forms may also comprise some additives, such as wetting agents, suspending agents, emulsifying agents, sweetening agents, flavoring agents, aroma, preservative or buffering agent.

In the parenteral administration, formulation of external use include, for example, ointment, gel, cream, poultice, patch, liniment, atomized agent, inhalation, spray, aerosol, eye drops and nasal spray, *etc*. They includes one or more of the active compound(s) and be prepared by known method or usual method.

Ointment is prepared by known method or usual method. For example, it is prepared by levigation or fusion of one or more of the active compound(s) and substrate. The substrate of ointment is selected from known or usual one. For example, higher fatty acid or higher fatty acid ester (adipic acid, myristic acid, palmitic acid, stearic acid, oleic acid, adipic acid ester, myristic acid ester, palmitic acid ester, stearic acid ester, oleic acid ester, *etc*.), wax (yellow beeswax, Spermaceti, ceresin, *etc*.), surfactant (polyoxyethylene alkyl ether phosphoric acid ester, *etc*.), higher alcohol (cetanol, stearil alcohol, cetostearyl alcohol, *etc.),* silicon oil (dimethyl polysiloxane, *etc*.), hydrocarbon (hydrophilic petrolatum, white petrolatum, purified lanolin, light liquid paraffin, *etc*.), glycol (ethylene glycol, diethylene glycol, propylene glycol, polyethylene glycol, macrogol, *etc*.), vegetable oil (castor oil, olive oil, sesame oil, turpentine oil, *etc.),* animal oil (mink oil, egg yolk oil, squalane, squalene, *eti*c.), water, absorption accelerator, skin fit inhibitor, *etc.* are used as single substance selected from them or mixture which consists of two or more kinds that is selected from them. Moreover, humectant, preservative agent, stabilizer, antioxidative agent, fragrant materials, *etc*. may be contained.

Ger is prepared by known method or usual method. For example, it is prepared by fusion of one or more of the active compound(s) and substrate. The substrate of gel is selected from known or usual one. For example, lower alcohol (ethanol, isopropylalcohol, *etc*.), gelling agent (carboxy methyl cellulose, hydroxy ethyl cellulose, hydroxy propyl cellulose, ethyl cellulose, *etc*.), neutralizing agent, (triethanolamine, diisopropanolamine, *etc.),* surfactant, (polyethylene glycol monostearate, *etc*.), gum, water, absorption accelerator, skin fit inhibitor, *etc*. are used as single substance selected from them or mixture which consists of two or more kinds that is selected from them. Moreover, preservative agent, antioxidative agent, fragrant materials, *etc*. may be contained.

Cream is prepared by known method or usual method. For example, it is prepared by fusion or emulsification of one or more of the active compound(s) and substrate. The substrate of cream is selected from known or usual one. For example, higher fatty acid ester, lower alcohol, hydrocarbon, polyalcohol (propylene glycol, 1,3-butylene glycol, *etc*.), higher alcohol (2-hexyldecanol, cetanol, *etc*.), emulsifying agent (polyoxyethylene anlyl ether, fatty acid ester, *etc*.), water, absorption accelerator, skin fit inhibitor, *etc.* are used as single substance selected from them or mixture which consists of two or more kinds that is selected from them. Moreover, preservative agent, antioxidative agent, fragrant materials, *etc*. may be contained.

Poultice is prepared by known method or usual method. For example, it is prepared by fusion of one or more of the active compound(s) and substrate, and then the kneaded one is laid over support medium. The substrate for poultice is selected from known or usual one. For example, thickening agent (polyacrylic acid, polyvinylpyrolidone, gum acacia, starch, gelatin, methyl cellulose, *etc*.), bulking agent (kaolin, zinc oxide, talc, calcium, magnesium, *etc*.), water, solubilizing agent, thickener, skin fit inhibitor, *etc*. are used as single substance selected from them or mixture which consists of two or more kinds that is selected from them. Moreover, preservative agent, antioxidative agent, fragrant materials, *etc*. may be contained.

Patch is prepared by known method or usual method. For example, it is prepared by fusion of one or more of the active compound(s) and substrate, and then laid over support medium. The substrate for patch is selected from known or usual one. For example, polymer substrate, fat, higher fatty acid, thickener, skin fit inhibitor, *etc*. are used as single substance selected from them or mixture which consists of two or more kinds that is selected from them. Moreover, preservative agent, antioxidative agent, fragrant materials, *etc*. may be contained.

Liniment is prepared by known method or usual method. For example, one or more of the active compound(s) may be dissolved, suspended or emulsified in water, alcohol (ethanol, polyethylene glycol, *etc.),* higher fatty acid, glycerin, soap, emulsifying agent, suspending agent, *etc.* as single substance selected from them or mixture which consists of two or more kinds that is selected from them. Moreover, preservative agent, antioxidative agent, fragrant materials, *etc*. may be contained.

Atomized agent, inhalation and spray may comprise in addition to a diluent, a stabilizer such as sodium bisulfite and an isotonization buffer such as sodium chloride, sodium citrate or citric acid. The preparation process of sprays is described in detail in, for example, U.S. Patent Nos. 2,868,691 and 3,095,355.

Injections for parenteral administration include sterile aqueous, suspensions, emulsions and solid forms which are dissolved or suspended into solvent(s) for injection immediately before use. In injections, one or more of the active compound(s) may be dissolved, suspended or emulsified into solvent(s). The solvents may include distilled water for injection, physiological salt solution, vegetable oil, propylene glycol, polyethylene glycol, alcohol, e.g. ethanol, or a mixture thereof Injections may comprise some additives, such as stabilizing agents, solution adjuvants (such as glutamic acid, aspartic acid or POLYSORBATE80 (registered trade mark)), suspending agents, emulsifying agents, soothing agent, buffering agents, preservative.
They may be sterilized at a final step, or may be prepared by an aseptic manipulation. They may also be manufactured in the form of sterile solid forms, for example, freeze-dried products, which may be dissolved in sterile water or some other sterile diluent(s) for injection immediately before use.

The dosage of inhalations for parenreral administration include aerosol, powders for inhalation or liquids for inhalation. The liquids for inhalation may be dissolved or suspended in water or the other appropriate solvent as needed.

Such inhalations are prepared in a known method.

For example, a liquid for inhalation is prepared by selecting proper additives from an antiseptic (such as benzalkonium chloride or p-aminobenzonic acid), a coloring agent, a buffering agent (such as sodium phosphate or sodium acetate), an isotonizing agent (such as sodium chloride or concentrated glycerin), thickening agent (such as carboxyvinylpolymer), or an accelerator of absorption, *etc.,* if necessary.

A powder for inhalation is prepared by selecting proper additives from a lubricant agent (such as stearin acid and the salt thereof), a binding agent, (such as starch, dextrin), a diluting agent (such as lactose, cellulose), a coloring agent, an antiseptic (such as benzalkonium chloride or p-aminobenzonic acid), an accelerator of absorption, *etc.,* if necessary.

In case of administration of liquid for inhalation, spray (atomizer, nebulizer) is usually used and in case of administration of powder for inhalation, inhalation administration apparatus for powder agents is usually used.

The other compositions for parenteral administration include suppositories for intrarectal administration and pessaries for vaginal administration which comprise one or more of the active substance(s) and may be prepared by methods known per se.

### BRIEF DESCRIPTTON OF THE DRAWINGS

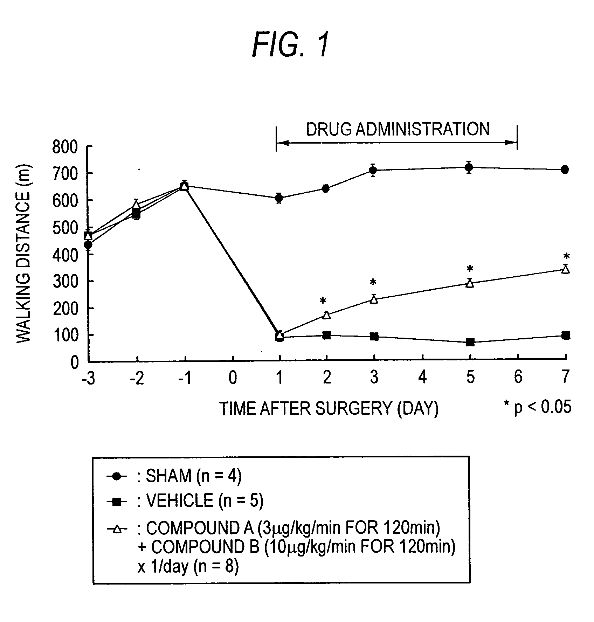

Fig. 1 shows that administration of the compound A ((5Z,9β,11α,13E)-17,17-propano-11,16-dihydroxy-9-chloro-20-norprosta-5,13-dienoic acid lysine salt) and the compound B (11α,15α-dimethoxy-9-oxoprosta-5Z,13E-dienoic acid) is improved the pathology in a rat gait disorder model induced by compression of cauda equina.

### BEST MODE FOR CARRYING OUT THE INVENTION

The present invention is explained below in detail based on Examples and Formulation Examples, but the present invention is not limited thereto.

### Example 1

### Improvement effect of the compound of the present invention in a rat gait disorder model induced by compression of cauda equina

### <Method of making an animal model>

A rat gait disorder model induced by compression of cauda equina was made by the method of Takenobu *et al*. (*J. Neurosci. Methods*, 104(2), 191-198 (2002)). Namely, a rat was anesthetized by sodium pentobarbital, removed its dorsal hair and then was fixed its body in the prone position. After disinfection of the back with Chlorhexidine gluconate (5% Hibiten Liquid: Sumitomo Pharmaceuticals), the lumbar was incised along the midline to expose the spine. After excision of the fifth lumbar spinous process, silicon rubber 1 × 4 × 1.25 mm (height × length × width) were inserted into the fourth and the sixth lumbar spinal canals from small holes of vertebral arch which was made by mini-drill. Benzylpenicillinpotassium (penicellin G potassium Meiji; Meiji Seika) was dropped into the incised part and injected into femor muscle. Muscle and skin of the incised part were closed by surgical suture. Sutured part was painted with iodine tincture. A sham-operated rat was made by the above described method except for the insertion of silicon rubber.

### <Examination of walking ability>

The walking ability was examined by using the treadmill apparatus.

The rats were put on the running belt, and adapted to the condition where the grid was sent an electric current (0.04mA - 4mA) for three minutes or more. Animals were forced to walk by an initial speed of 10 m/min, which was gradually increased by 5 m/min at 3 min intervals. Electric stimulation (0.04mA - 4mA) was given to the rats that stopped walking and moved to the grid for electric stimulation equipped in front of the running belt. The distance between the point to walk and the point to give up walk, in other words, the point where it is impossible for them to walk even though the stimulation (sound, contact and electricity) to make them walk was given, was measured with the mileometer built into the equipment. The walking training was performed once a day for three days before the operation. After the operation, (5Z,9β,11α,13E)-17,17-propano-11,16-dihydroxy-9-chloro-20-norprosta-5,13-dienoic acid lysine salt (the compound (A)) having EP2 agonist action and 11α,15α-dimethoxy-9-oxoprosta-5Z,13E-dienoic acid (the compound (B)) having EP3 agonist action were administered. On the other hand, saline was administered as a negative control. The results obtained from the compounds- and negative control-groups were analyzed by the Dunnett's multiple comparison test (* p<0.05).

### <Result>

The gait disorder model induced by compression of cauda equina is reported as a model for the spinal canal stenosis. The combination of the compound A and the compound B improved the gait disorder of rat induced by compression of cauda equina. That is, it was indicated that the activation of EP2 receptor and EP3 receptor could be effective for the treatment for spinal canal stenosis. Therefore, the combination of the compound having EP2 agonist action and the compound having EP3 agonist action, or the compound having EP2 agonist action and EP3 agonist action can prevent and/ or treat for spinal canal stenosis. Result is shown at figure 1.

### Formulation Example 1

The following components were admixed in a conventional method and punched out to obtain 100,000 tablets each containing 0.5 mg (the compound A : 0.12 mg, the compound B : 0.38 mg) of the active ingredient.

| | |
|---|---|
| Compound A | 12 g |
| Compound B | 38 g |
| Carboxymethyl cellulose calcium | 200 g |
| Magnesium stearate | 100 g |
| Microcrystalline cellulose | 9.2 kg |

### Formulation Example 2 :

The following components were admixed in a conventional method, and the solution was sterilized in a conventional method, placed at 1 ml into vials and freeze-dried in a conventional method to thereby obtain 100,000 vials each containing 0.2 mg (the compound A: 0.05 mg, the compound B : 0.15 mg) of the active ingredient.

| | |
|---|---|
| Compound A | 5 g |
| Compound B | 15 g |
| Mannitol | 5 kg |
| Distilled water | 100 L |

### Formulation Example 3 :

The following components were admixed in a conventional method and punched out to obtain 100,000 tablets each containing 0.5 mg of the active ingredient.

| | |
|---|---|
| Compound A | 50 g |
| Carboxymethyl cellulose calcium | 200 g |
| Magnesium stearate | 100 g |
| Microcrystalline cellulose | 9.2 kg |

### Formulation Example 4 :

The following components were admixed in a conventional method, and the solution was sterilized in a conventional method, placed at 1 ml into vials and freeze-dried in a conventional method to thereby obtain 100,000 vials each containing 0.2 mg of the active ingredient.

| | |
|---|---|
| Compound A | 20 g |
| Mannitol | 5 kg |
| Distilled water | 100 L |

### Formulation Example 5 :

The following components were admixed in a conventional method and punched out to obtain 100,000 tablets each containing 0.5 mg of the active ingredient.

| | |
|---|---|
| Compound B | 50 g |
| Carboxymethyl cellulose calcium | 200 g |
| Magnesium stearate | 100 g |
| Microcrystalline cellulose | 9.2 kg |

### Formulation Example 6 :

The following components were admixed in a conventional method, and the solution was sterilized in a conventional method, placed at 1 ml into vials and freeze-dried in a conventional method to thereby obtain 100,000 vials each containing 0.2 mg of the active ingredient.

| | |
|---|---|
| Compound B | 20 g |
| Mannitol | 5 kg |
| Distilled water | 100 L |

### Formulation Example 7 :

100,000 tablets each containing 1 mg (the compound A : 0.24 mg, the compound B : 0.76 mg) of the active ingredient were obtained by the same method as Formulation Example 1.

| | |
|---|---|
| Compound A | 24 g |
| Compound B | 76 g |
| Carboxymethyl cellulose calcium | 200 g |
| Magnesium stearate | 100 g |
| Microcrystalline cellulose | 9.2 kg |

### Formulation Example 8 :

100,000 vials each containing 1 mg (the compound A : 0.25 mg, the compound B : 0.75 mg) of the active ingredient were obtained by the method as Formulation Example 2.

| | |
|---|---|
| Compound A | 25 g |
| Compound B | 75 g |
| Mannitol | 5 kg |
| Distilled water | 100 L |

## Claims

1. An agent for preventing and/or treating spinal canal stenosis which comprises a combination of a compound having EP2 agonist action and a compound having EP3 agonist action.

2. The agent for preventing and/or treating spinal canal stenosis according to claim 1, wherein the compound having EP2 agonist action and the compound having EP3 agonist action are each administrated.

3. The agent for preventing and/or treating spinal canal stenosis according to claim 1, wherein the compound having EP2 agonist action and the compound having EP3 agonist action are comprised in the same preparation.

4. The agent for preventing and/or treating spinal canal stenosis according to claim 1, wherein the compound having EP2 agonist action is a compound represented by formula (I) 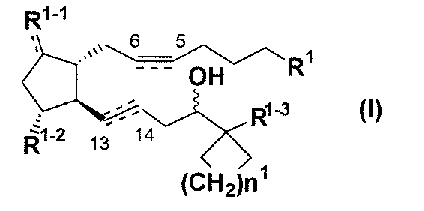 wherein R¹ is carboxy or hydroxymethyl; R¹⁻¹ is oxo, methylene or a halogen atom; R¹⁻² is a hydrogen atom, hydroxy or C1-4 alkoxy; R¹⁻³ is a hydrogen atom, C1-8 alkyl, C2-8 alkenyl, C2-8 alkynyl, or C1-8 alkyl, C2-8 alkenyl or C2-8 alkynyl substituted by 1-3 of substituents selected from the following (1) to (5): (1) a halogen atom, (2) C1-4 alkoxy, (3) C3-7 cycloalkyl, (4) phenyl or (5) phenyl substituted by 1-3 of substituents selected from a halogen atom, C1-4 alkyl, C1-4 alkoxy, nitro or trifluoromethyl; n¹ is 0 or 1-4; 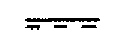 is a single bond or a double bond; is a double bond or a triple bond; 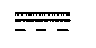 is a single bond, a double bond or a triple bond; 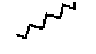 is α-configuration, β-configuration or a mixture of them,
a salt thereof, a solvate thereof or a prodrug thereof, or a cyclodextrin clathrate thereof.

5. The agent for preventing and/or treating spinal canal stenosis according to claim 1, wherein the compound having EP3 agonist action is a compound represented by formula (II) 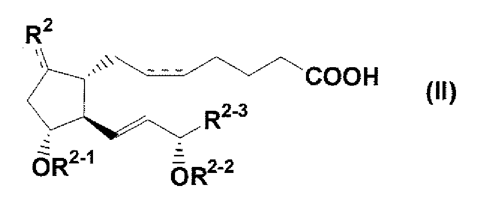 wherein R² is oxo or a halogen atom; R²⁻¹ and R²⁻² are each independently C1-4 alkyl; R²⁻³ is C1-10 alkyl, C2-10 alkenylene, C2-10 alkynylene, or C1-10 alkyl, C2-10 alkenylene, C2-10 alkynylene substituted by phenyl, phenoxy, C3-7 cycloalkyl or C3-7 cycloalkyloxy, in which the phenyl and the cycloalkyl may be substituted by 1-3 of C1-4 alkyl, C1-4 alkoxy, a halogen atom, trihalomethyl or nitro; 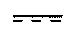 is a single bond or a double bond,
a salt thereof, a solvate thereof or a prodrug thereof, or cyclodextrin clathrate thereof.

6. The agent for preventing and/or treating spinal canal stenosis according to claim 1, wherein the compound having EP2 agonist action is a compound represented by formula (III) 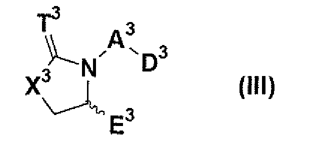 wherein T³ is (1) an oxygen atom or (2) a sulfur atom;
X³ is (1) -CH₂-, (2) -O- or (3) -S-;
A³ is A³⁻¹ or A³⁻²; A³⁻¹ is (1) C2-8 straight-chain alkylene optionally substituted by 1 to 2 C1-4 alkyl, (2) C2-8 straight-chain alkenylene optionally substituted by 1 to 2 C1-4 alkyl or (3) C2-8 straight-chain alkynylene optionally substituted by 1 to 2 C1-4 alkyl; A³⁻² is -G³⁻¹-G³⁻²-G³⁻³-; G³⁻¹ is (1) C1-4 straight-chain alkylene optionally substituted by 1 to 2 C1-4 alkyl, (2) C2-4 straight-chain alkenylene optionally substituted by 1 to 2 C1-4 alkyl or (3) C2-4 straight-chain alkynylene optionally substituted by 1 to 2 C1-4 alkyl; G³⁻² is (1) -Y³-, (2) -ring 1³-, (3) -Y³-ring 1³-, (4) -ring 1³-Y³- or (5) -Y³-C1-4 alkylene-ring 1³-; Y³ is (1) -S-, (2) -SO-, (3) -SO₂-, (4) - O- or (5) -NR³⁻¹-; R³⁻¹ is (1) a hydrogen atom, (2) C1-10 alkyl or (3) C2-10 acyl; G³⁻³ is (1) a bond, (2) C1-4 straight-chain alkylene optionally substituted by 1 to 2 C1-4 alkyl, (3) C2-4 straight-chain alkenylene optionally substituted by 1 to 2 C1-4 alkyl or (4) C2-4 straight-chain alkynylene optionally substituted by 1 to 2 C1-4 alkyl;
D³ is D³⁻¹ or D³⁻²; D³⁻¹ is (1) -COOH, (2) -COOR³⁻², (3) tetrazol-5-yl or (4) - CONR³⁻³SO₂R³⁻⁴; R³⁻² is (1) C1-10 alkyl, (2) phenyl, (3) C1-10 alkyl substituted by phenyl or (4) biphenyl; R³⁻³ is (1) a hydrogen atom or (2) C1-10 alkyl; R³⁻⁴ is (1) C1-10 alkyl or (2) phenyl; D³⁻² is (1) -CH₂OH, (2) -CH₂OR³⁻⁵, (3) hydroxy, (4) -OR³⁻⁵, (5) formyl, (6) -CONR³⁻⁶R³⁻⁷, (7) -CONR³⁻⁶SO₂R³⁻⁸, (8) -CO-(NH-amino acid residue-CO)ₘ₃-OH, (9)-O-(CO-amino acid residue-NH)ₘ₃-H, (10) -COOR³⁻⁹, (11) -OCO-R³⁻¹⁰, (12) -COO-Z³⁻¹-Z³⁻²-Z³⁻³ or 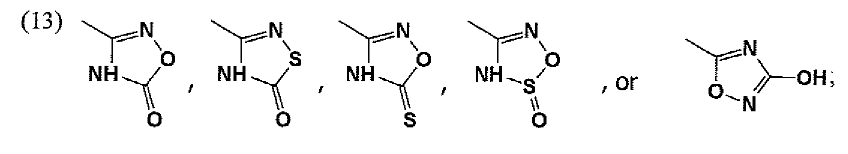 R³⁻⁵ is C1-10 alkyl; R³⁻⁶ and R³⁻⁷ are each independently (1) a hydrogen atom or (2) C1-10 alkyl; R³⁻⁸ is C1-10 alkyl substituted by phenyl; R³⁻⁹ is (1) C1-10 alkyl substituted by biphenyl optionally substituted by 1 to 3 substituents selected from C1-10 alkyl, C1-10 alkoxy and a halogen atom or (2) biphenyl substituted by 1 to 3 substituents selected from C1-10 alkyl, C1-10 alkoxy and a halogen atom; R³⁻¹⁰ is (1) phenyl or (2) C1-10 alkyl; m³ is 1 or 2; Z³⁻¹ is (1) C1-15 alkylene, (2) C2-15 alkenylene or (3) C2-15 alkynylene; Z³⁻² is (1) -CO-, (2) -OCO-, (3) -COO-, (4) -CONR^{Z3-1}-, (5) -NR^{Z3-2}CO-, (6) -O-, (7) -S-, (8) -SO₂-, (9) -SO₂-NR^{Z3-2}-, (10) -NR^{Z3-2}SO₂-, (11) -NR^{Z3-3}-, (12) -NR^{Z3-} ⁴CONR^{Z3-5}-, (13) -NR^{Z3-6}COO-, (14) -OCONR^{Z3-7}- or (15) -OCOO-; Z³⁻³ is (1) a hydrogen atom, (2) C1-15 alkyl, (3) C2-15 alkenyl, (4) C2-15 alkynyl, (5) ring Z³ or (6) C1-10 alkyl substituted by C1-10 alkoxy, C1-10 alkylthio, C1-10 alkyl-NR^{Z3-8}- or ring Z³; ring Z³ is (1) C3-15 mono-, bi- or tri-carbocyclic aryl which may be partially or fully saturated or (2) 3 to 15 membered mono-, bi- or tri-heterocyclic aryl containing 1 to 4 hetero atoms selected from oxygen, nitrogen and sulfur atom which may be partially or fully saturated; R^{Z3-1}, R^{Z3-2}, R^{Z3-3}, R^{Z3-4}, R^{Z3-5}, R^{Z3-6}, R^{Z3-7} and R^{Z3-8} are each independently a hydrogen atom or C1-15 alkyl, R^{Z3-1} and Z³⁻³ may be taken together with the nitrogen atom to which they are attached to form 5 to 7 membered saturated mono-heterocyclic ring, and the heterocyclic ring may contain other one hetero atom selected from oxygen, nitrogen and sulfur atoms, ring Z³ and the saturated mono-heterocyclic ring formed by R^{Z3-1}, Z³⁻³ and the nitrogen atom to which they are attached may be substituted by 1-3 groups selected from following (1) to (4); (1) C1-15 alkyl, (2) C2-15 alkenyl, (3) C2-15 alkynyl, (4) C1-10 alkyl substituted by C1-10 alkoxy, C1-10 alkylthio or C1-10 alkyl-NR^{Z3-9}-; R^{Z3-9} is a hydrogen atom or C1-10 alkyl, 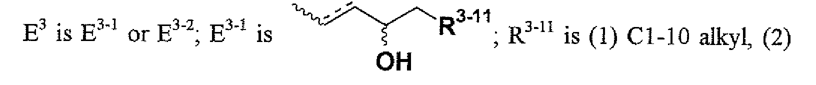 C1-10 alkylthio, (3) C1-10 alkyl substituted by C3-8 cycloalkyl, (4) C1-10 alkyl substituted by ring 2 or (5) C1-10 alkyl substituted by -W³⁻¹-W³⁻²-ring 2; W³⁻¹ is (1) -O-, (2) -S-, (3) -SO-, (4) -SO₂-, (5) -NR³⁻¹¹⁻¹-, (6) carbonyl, (7) -NR³⁻¹¹⁻¹SO₂-, (8) carbonylamino or (9) aminocarbonyl; R³⁻¹¹⁻¹ is (1) a hydrogen atom, (2) C1-10 alkyl or (3) C2-10 acyl; W³⁻² is (1) a bond or (2) C1-8 alkyl optionally substituted by C1-4 alkyl, a halogen atom or hydroxy; E³⁻² is (1) U³⁻¹-U³⁻²-U³⁻³ or (2) ring 4³; U³⁻¹ is (1) C1-4 alkylene, (2) C2-4 alkenylene, (3) C2-4 alkynylene, (4) -ring 3³-, (5) C1-4 alkylene-ring 3³-, (6) C2-4 alkenylene-ring 3³- or (7) C2-4 alkynylene-ring 3³-; U³⁻² is (1) a bond, (2) -CH₂-, (3) -CHOH-, (4) -O-, (5) -S-, (6) -SO-, (7) -SO₂-, (8) -NR³⁻¹²-, (9) carbonyl, (10) -NR³⁻¹²SO₂-, (11) carbonylamino or (12) aminocarbonyl; R³⁻¹² is (1) a hydrogen atom, (2) C1-10 alkyl or (3) C2-10 acyl; U³⁻³ is (1) C1-8 alkyl optionally substituted by 1 to 3 substituents selected from C1-10 alkyl, a halogen atom, hydroxy, alkoxy, alkylthio and NR³⁻¹³R³⁻¹⁴, (2) C2-8 alkenyl optionally substituted by 1 to 3 substituents selected from C1-10 alkyl, a halogen atom, hydroxy, alkoxy, alkylthio and NR³⁻¹³R³⁻¹⁴, (3) C2-8 alkynyl optionally substituted by 1 to 3 substituents selected from C1-10 alkyl, a halogen atom, hydroxy, alkoxy, alkylthio and NR³⁻¹³R³⁻¹⁴, (4) C1-8 alkyl substituted by ring 4³ or (5) ring 4³; R³⁻¹³ and R³⁻¹⁴ are each independently (1) a hydrogen atom or (2) C1-10 alkyl; ring 1³, ring 2³, ring 3³ or ring 4³ may be substituted by 1 to 5 of R³; R³ is (1) C1-10 alkyl, (2) C2-10 alkenyl, (3) C2-10 alkynyl, (4) C1-10 alkoxy, (5) C1-10 alkylthio, (6) a halogen atom, (7) hydroxy, (8) nitro, (9) -NR³⁻¹⁵R³⁻¹⁶, (10) C1-10 alkyl substituted by C1-10 alkoxy, (11) C1-10 alkyl substituted by 1 to 3 halogen atoms, (12) C1-10 alkyl substituted by C1-10 alkoxy substituted by 1 to 3 halogen atoms, (13) C1-10 alkyl substituted by -NR³⁻¹⁵R³⁻¹⁶, (14) ring 5³, (15) -O-ring 5³, (16) C1-10 alkyl substituted by ring 5³, (17) C2-10 alkenyl substituted by ring 5³, (18) C2-10 alkynyl substituted by ring 5³, (19) C1-10 alkoxy substituted by ring 5³, (20) C1-10 alkyl substituted by -O-ring 5³, (21) COOR³⁻¹⁷, (22) C1-10 alkoxy substituted by 1 to 4 halogen atom, (23) formyl, (24) C1-10 alkyl substituted by hydroxy or (25) C2-10 acyl, R³⁻¹⁵; R³⁻¹⁶ and R³⁻¹⁷ are each independently (1) a hydrogen atom or (2) C1-10 alkyl; ring 5³ may be substituted by 1 to 3 substituents selected from following (1)-(9); (1) C1-10 alkyl, (2) C2-10 alkenyl, (3) C2-10 alkynyl, (4) C1-10 alkoxy, (5) C1-10 alkyl substituted by C1-10 alkoxy, (6) a halogen atom, (7) hydroxy, (8) C1-10 alkyl substituted by 1 to 3 halogen atoms, (9) C1-10 alkyl substituted by C1-10 alkoxy substituted by 1 to 3 halogen atoms; ring 1³, ring 2³, ring 3³, ring 4³ and ring 5³ are each independently (1) C3-15 mono-, bi- or tri-carbocyclic aryl which may be partially or fully saturated or (2) 3 to 15 membered mono-, bi- or tri-heterocyclic aryl containing hetero atoms selected from 1 to 4 nitrogen, 1 to 2 oxygen and/or 1 to 2 sulfur atom which may be partially or fully saturated; 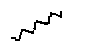 is α-configuration, β-configuration or mixture of them,
a salt thereof, a solvate thereof or a prodrug thereof, or cyclodextrin clathrate thereof.

7. An agent for preventing and/or treating spinal canal stenosis which comprises a compound having EP2 agonist action and EP3 agonist action.

8. The agent for preventing and/or treating spinal canal stenosis according to claim 1 or 7, wherein the spinal canal stenosis is cervical spinal canal stenosis, thoracic spinal canal stenosis, lumbar spinal canal stenosis or wide spinal canal stenosis.

9. The agent for preventing and/or treating spinal canal stenosis according to claim 1 or 7, which is an agent for improving paralysis, hypoesthesia, pain or numbness.

10. The agent for preventing and/or treating spinal canal stenosis according to claim 1 or 7, which is an agent for improving physical ability.

11. The agent for preventing and/or treating spinal canal stenosis according to claim 10, wherein the improving physical ability is improving muscle weakness, intermittent claudication or ambulatory ability.

12. The agent for preventing and/or treating spinal canal stenosis according to claim 1 or 7, which is an agent for treating bladder trouble or rectum trouble.

13. A medicament which comprises a combination of the agent for preventing and/or treating spinal canal stenosis according to claim 1 or 7 and one or more medicaments selected from prostaglandins, prostaglandin derivatives formulations, nonsteroidal anti-inflammatory drugs, vitamins, muscle relaxants, antidepressants, poly ADP-ribose polymerase inhibitors, excitatory amino acid receptor antagonists, radical scavengers, astrocyte modulators, IL-8 receptor antagonists, immunosuppressive drugs, nitric oxide synthase inhibitor and aldose reductase inhibitors.

14. A method for preventing and/or treating spinal canal stenosis in a mammal, which comprises administering to a mammal an effective amount of a compound having EP2 agonist action and a compound having EP3 agonist action, or a compound having EP2 agonist action and EP3 agonist action.

15. Use of a compound having EP2 agonist action and a compound having EP3 agonist action, or a compound having EP2 agonist action and EP3 agonist action for preparation of an agent for preventing and/or treating spinal canal stenosis.
